**Europäisches Patentamt**

**European Patent Office**

**Office européen des brevets**

(19)

(11) Publication number: **0 250 234**
**A1**

(12) # EUROPEAN PATENT APPLICATION

(21) Application number: **87305389.6**

(22) Date of filing: **17.06.87**

(51) Int. Cl.⁴: **A 61 K 45/05,** A 61 K 35/14, A 61 K 39/00, A 61 K 35/26, C 12 N 5/00

(30) Priority: **17.06.86 GB 8614733**

(43) Date of publication of application: **23.12.87** Bulletin 87/52

(84) Designated Contracting States: **AT BE CH DE ES FR GB GR IT LI LU NL SE**

(71) Applicant: **NEWTHERAPEUTICS LIMITED, 10 John Street, London WC1N 2EB (GB)**

(72) Inventor: **Lefesvre, Andre, 3 bis Rue du General Leclerc, F-78110 Le Vesinet (FR)**

(74) Representative: **Woods, Geoffrey Corlett et al, J.A. KEMP & CO. 14 South Square Gray's Inn, London WC1R 5EU (GB)**

(54) **Use of specific immunomodulators in treating viral-induced immunodeficiencies.**

(57) A product for use in combating an infection such as AIDS attributable to a retrovirus is prepared by extracting cells of lymphoid origin and/or liver cells from an animal or human immunised with respect to the retrovirus, disrupting the cells and dialysing or filtering the contents of the cells thus-released thereby to obtain a retrovirus-specific transfer factor.

EP 0 250 234 A1

DESCRIPTION

USE OF SPECIFIC IMMUNOMODULATORS IN TREATING

VIRAL-INDUCED IMMUNODEFICIENCIES

This invention relates to the treatment of viral infections attributable to retroviruses. More specifically, it relates to the use of dialysable leucocyte extract (DLE) (transfer factor) to treat AIDS patients.

At present, AIDS is a fatal immunological disorder caused by a lymphotropic retrovirus. In 1984, three research groups reported independently that they had sequenced the entire genome of the retrovirus. Each group called its virus by a different name - human T-lymphotropic virus III (HTLV-III), lymphoadenopathy/AIDS virus (LAV) and AIDS-associated retrovirus (ARV). It has not been unequivocally established that the groups were studying the same virus. Indeed, a large number of genetic variants have been isolated from patients. In this specification, the generic term Human Immunodeficiency Virus (HIV) is used.

Various antiviral therapies including suramin, heteropolyanion-23 (HPA-23), ribavirin and $\alpha$-interferon have been tried to treat AIDS patients but all have failed to produce the expected clinical effects. 3-Azido-3'--deoxythymidine is the only antiviral agent which has shown some promising results. In view of these findings, alternative methods of treatment of AIDS patients have been sought and immune stimulation has therefore been tried.

- 2 -

However, disappointing results have been obtained. Cimetidin, indomethacin, thymosin fraction S. and inosine pranobex or isoprinosine have all been used but with no promising results.

It has now been found that the use of HIV-specific transfer factor can result in a marked improvement in the clinical condition of AIDS patients with increase or at least stabilisation of their T4 lymphocyte sub-population. Administration of the transfer factor may be accompanied by administration also of one or more of thymic and/or spleen and/or liver extracts, anabolites and antiviral drugs.

This approach is applicable to the treatment of viral infections attributable to retroviruses generally. Accordingly, the present invention provides a process for the preparation of a product for use in combating an infection attributable to a retrovirus, which process comprises extracting cells of lymphoid origin and/or liver cells from an animal or human immunised with respect to the retrovirus, disrupting the cells and dialysing or filtering the contents of the cells thus-released thereby to obtain a retrovirus-specific transfer factor.

The invention also provides a product suitable for use in combating an infection attributable to a retrovirus, which product comprises a retrovirus-specific transfer factor prepared from cells of lymphoid origin and/or liver cells of an animal or human previously

- 3 -

immunised with respect to the retrovirus.

Also provided is a product containing

(a) a retrovirus-specific transfer factor prepared from cells of lymphoid origin and/or liver cells of an animal or human previously immunised with respect to the retrovirus and

(b) one or more selected from thymic and/or spleen and/or liver extracts, anabolites and antiviral drugs
for simultaneous or sequential use in combating an infection attributable to a retrovirus in a human or animal.

The retrovirus-specific transfer factor may be prepared in an appropriate manner. Any suitable animal, for example guinea pigs or mice, can be immunised. The retrovirus may be HTLV-I, HTLV-II or a Lentivirus such as HIV-1, HIV-2, SIV or medi-visna. Preferably, the animal is healthy and does not show a positive reaction for the virus when tested prior to immunisation. Typically, several injections of fresh virus-infected lymphocytes are given to an animal over a period of time. Alternatively, virus-infected lymphoblastoid cells, virus itself or viral antigens may be used for immunisation. The point at which the animal has acquired the necessary degree of immunisation can be evaluated by, for example, a skin test, leucocyte migration inhibition test and/or lymphocyte transformation test.

- 4 -

The retrovirus-specific transfer factor may be obtained from cells of lymphoid origin and/or the liver cells of the immunised animal or human. By an "immunised" human is meant a human seropositive for the virus or a human positive for the virus itself. A human may be immunised by appropriate presentation of the virus, e.g. by vaccination. Alternatively, it may be found that a patient is naturally seropositive or positive for the virus. The cells of lymphoid origin may be peripheral blood lymphocytes or cells from lymphatic tissues such as the spleen or lymph nodes. A suppressor lymphocyte sub-population, T8 lymphocytes in the case of humans, may be separated and used. The transfer factor is obtained from the cells, extracted from the immunised mammal, by disrupting the cells thereby to release their contents and dialysing their contents. The cells may be disrupted by repeated freeze-thawing, by ultra-sonication or by mechanical means such as in a mixer. Typically, the disrupted cells are dialysed through a dialysis bag (Visking cellulose) against distilled water or a vacuum. Alternatively, the disrupted cells can be introduced into an ultra-filtration apparatus possessing, for example, an Amicon filter. The dialysate or filtrate thus obtained comprises active moieties known as transfer factor or DLE specific to the virus.

The dialysate comprises retrovirus-specific inducer (helper) factor and a suppressor factor capable of inducing immunosuppression or immunoregulation of the immune response to the retrovirus. It has been found that transfer factor prepared from suppressor lymphocytes, i.e. those associated with the suppressor factor, can be used to improve the clinical condition of patients whereas transfer factor derived from helper lymphocytes does not. Transfer factor derived from suppressor lymphocytes may be prepared by:

1) after obtaining the cells of lymphoid origin, removing the T-helper (T4) cells or collecting the T-suppressor (T8) cells, and then obtaining a cell fraction, for example by dialysis; or

2) obtaining the desired cell fraction from the cells of lymphoid origin or liver cells and either removing the helper cell fraction from the cell fraction or collecting the suppressor moiety from the cell fraction, for example by immunoadsorption of the suppressor factor and subsequent elution of the suppressor factor.

It is also possible to use retrovirus-specific transfer factor replicated by a lymphoblastoid cell line as described in GB-A-1443948. A preferred cell line is the LDV/7 cell line described in GB-A-1592954. The lymphoblastoid cell line may be induced either by retrovirus-specific transfer factor. In the case of transfer factor obtained from AIDS patients, such individuals may either be without clinical symptoms of AIDS or be in remission. Again, the HIV-specific transfer factor with which the cell line is incubated may be a conventional total extract or have been obtained from a T8 lymphocyte sub-population. The cultured cells or the cells carrying a suppressor (T8) marker are disrupted and transfer factor obtained from the cell contents as above.

Whichever source of retrovirus-specific transfer factor is administered to a patient, the transfer factor is generally in the form of a cell fraction containing substantially no molecules with a molecular weight of more than 12,000. This ensures that the cell fraction does not contain any viral particles.

The transfer factor may be lyophilised for example for storage. When required for use, the lyophilised extract can be resuspended in a suitable volume of a physiologically acceptable diluent, for example saline, so as to form an isotonic solution. The invention therefore also provides a pharmaceutical composition comprising as active ingredient, retrovirus-specific transfer factor together with a physiologically acceptable carrier or diluent.

The transfer factor is typically administered orally or by injection to a human patient or to an animal, typically to combat a retrovirus-induced immunodeficiency. It may be administered to a patient who is HIV-seropositive, in whom HIV has been detected, and/or who exhibits the clinical conditions of AIDS. However, it may also be administered preventatively to HIV-seropositive or HIV-seronegative patients. It can be administered to a group considered to be at particular risk such as homosexuals or intravenous drug users. In this way, the transfer factor may be used to prevent evolution of AIDS-related complex and pre-AIDS condition.

Typically, the transfer factor is administered orally or by injection, for example subcutaneously, intravenously or intramuscularly. The amount of transfer factor administered will depend upon a variety of a factors, including the patient under treatment and the severity of the infection. Typically, the transfer factor may be administered at a dose of 0.1 to 100 units, preferably about 1 unit, for a suitable period of time. An amount of from 0.01 to 0.3, preferably from 0.1 to 0.3, mg may be administered. The transfer factor can be given daily or once a week. This is applicable regardless of the route of administration. One unit is, or corresponds to, the amount of transfer factor obtained by dialysis from $10^7$ cells.

The HIV-specific transfer factor may be administered in association with thymic and/or spleen and/or liver embryonic extracts, an anabolite and/or vitamins. The thymic, spleen or liver extract may be an extract from adult tissue but preferably is an embryonic extract. Such an extract, anabolite and/or vitamins may be given prior to, simultaneously with or subsequent to the transfer factor. The anabolite may be methenolone acetate. Thus, in addition to the transfer factor a patient may

- 8 -

receive one intramuscular injection of total embryonic
bovine thymic extract, 100 mg/day of methenolone acetate
orally and a poly-vitamin association.

The extract may be prepared by obtaining the
appropriate organ from an animal, for example a 4 month old
calf. The tissue is disrupted, typically minced. An Ultra
Turax apparatus may be used. The disrupted tissue is then
passed through a syringe to obtain a cell suspension which
may be stored frozen at -70°C. The cells may be
administered after resuspension in a physiologically
acceptable diluent, for example saline. The extract is
typically administered at $10^8$ to $10^9$ cells per injection,
intramuscularly.

The poly-vitamin association may be one which is
known and which contains the following vitamins:

| | |
|---|---|
| A | 12500 International Units (I.U.) |
| B1 | 5 mg |
| B2 | 4 mg |
| B5 | 10 mg |
| B6 | 5 mg |
| PP | 25 mg |
| C | 125 mg |
| D2 | 2500 I.U. |
| E | 5 mg |

Any antiviral drug may be administered in
association with the HIV-specific transfer factor. Again,
the drug may be administered prior to, simultaneously with

- 9 -

or subsequent to the transfer factor. The antiviral agent
may be anti-thymosin antibody, suramin, heteropolyanion-23
(HPA-23), ribavirin, $\alpha$-interferon or
3-azido-3'-deoxythymidine. Two or more such agents may be
used.

The antiviral agent may be one with a low
toxicity or may be used in non-toxic amounts. To achieve
this, the antiviral agent may be used in an amount of a
quarter to a third of its normal dose. In other words,
sufficient is administered not to produce side-effects.

The following Examples illustrate the invention.

Example 1:  Preparation of HIV (HIV-1) specific transfer
factor

Transfer factor was prepared by immunization of
10-14 week old Balb/c mice. The mice were given three
bi-weekly subcutaneous injections of $10^7$ lymphocytes, from
a pool obtained from four AIDS patients, mixed with 0.5 ml
complete Freund's Adjuvant. Transfer factor was extracted
from the spleen and livers of the mice three weeks after
the final immunization by dialysis using conventional
techniques which have been described elsewhere (Lawrence
H.S. "Transfer Factor in Cellular Immunity" The Harvey
Lectures (68th series), Academic Press, 1974, 239-350;
Pizza G. et al "In Vitro Production of a Transfer Factor
Specific for Translational Cell Carcinoma of the Bladder"
Br.J. Cancer 33, 1977, 606-611; Viza D. et al "Specific
Bovine Transfer Factor for the treatment of Herpes

- 10 -

Infections" Immunobiology of Transfer Factor (Kirkpatrick
et al eds.) Academic Press, 1983, 245-259). The dialysate
obtained (TFd) was HIV (HIV-1) -specific transfer factor.

Example 2:  Treatment of patient (1)

Patient DUR was a 41 year-old male homosexual.
He was first admitted for fevers, dyspnea due to
pneumocystis carinii, night-sweats, weight loss,
lymphadenopathy (5 cm) and fatigue to an infectious
diseases hospital.  AIDS was later diagnosed.  He was found
to have anti-HIV antibodies (anti-HIV-1 antibodies).  Under
antibiotic treatment dyspnea was reduced.  However dyspnea
and fever still persisted by the third month.  After
administration of sulphadiazine for two weeks, dyspnea
subsided and a slight clinical improvement was noted.  On
day 140 after admission he began standard TFd treatment
consisting of TFd administered orally at weekly intervals
at a dose of 2 x $10^7$ cell equivalent (c.equ.)/week and, in
addition, one intramuscular injection of total embryonic
bovine thymic extract at 5 x $10^8$ c.equ., 100 mg/day of
methenolone acetate orally and a poly-vitamin association
as described above.

The patient's condition improved thereafter:
dyspnea disappeared, his general well-being improved,
night-sweats disappeared, lymphadenopathy decreased to 1
cm.  Skin-tests to tetanus and steptococcus were very
slightly positive (0.5 mm diameter) but unfortunately the
patient was not skin-tested on admission.  At the end of

- 11 -

the six-month the clinical condition of the patient was markedly improved, he regained enough muscle strength to go on vacation. The number of T4 cells was normal as was the T4/T8 ratio. The results are shown in Table 1.

The patient was given the weekly standard TFd treatment for a further six months. Three months after this treatment stopped, his condition remained stable. No relapses had occurred. Lymphocyte cultures taken from the patient were found to be slightly positive for HIV three weeks before the completion of treatment. However, two weeks after completion of treatment cultures were negative. HIV infection was evaluated by reverse transcriptase assay.

Example 3:  Treatment of patient (2)

Patient PAU was a 30 year-old male homosexual. He was admitted suffering from fever (38°C), pseudo tumoral generalized lymphadenopathy, parotiditis and multiple facial "Kaposi-like" skin lesions (biopsy was not performed), weight loss and fatigue. He was found to be positive for HIV (HIV-1) antibodies, and the presence of virus was detected in the blood by co-culture. He was given sulphadiazine, sulphamethoxazole and trimethoprim. On day 35 extensive regression of lymphadenopathy was noted and temperature was normal; the patient was discharged. On day 92 the patient, coming for a check-up, had fever, extreme fatigue, loss of weight and moderate lymphadenopathy.

He began a 13 day TFd treatment on day 102.

- 12 -

Facial lesions had disappeared by day 121 and virus was not detected by co-culture on day 133. From day 147 the patient underwent further TFd treatment for 15 days. At the end of this period fever subsided and the patient had an improved sense of well-being. Standard TFd treatment began on the day 176. By day 198 the condition of the patient had further improved, with weight gain (+6 kg.) and total disappearance of adenopathy. T4 lymphocyte numbers and the T4/T8 ratio were normal. The patient has now resumed normal professional activity. The results are shown in Table 2.

The patient was given the weekly standard TFd treatment for a further six months. Three months after this treatment stopped, his condition remained stable. No relapses had occurred. Lymphocyte cultures from the patient showed HIV infection six weeks before the completion of treatment, as evaluated by reverse transcriptase assay. However, two weeks after completion of treatment cultures were negative for HIV.

Example 4: Treatment of patient (3)

Patient KER was a 45 year-old male homosexual. A year and a half before admission he had been diagnosed as having AIDS-related complex (ARC) and was treated by amoxyllin for bronchitis. He had fevers, lymphadenopathy and anti-HIV antibodies were detected in his serum. A year before admission he suffered from inflammatory polyradiculoneuropathy and biopsy showed diffuse lymphocyte

- 13 -

infiltration in the lungs, the peripheral nervous system and the mucosa of the digestive tract. When he was first admitted he weighed 54 kg. for a height of 180 cm. (a weight loss of 32 kg.), had lymphadenopathy, bronchitis, diarrhea, fevers (40°C), and purulent expectoration. He received sulphadiazine, sulphamethoxazole and trimethoprim.

He started TFd treatment at $10^7$ c.equ./day for 13 days on day 37 with a further course of treatment for 15 days starting on day 85. Standard TFd treatment began on day 120. During treatment, fevers decreased and there was general clinical improvement. He was re-admitted on day 131 for fatigue. By day 159 under continuous TFd treatment fatigue had subsided, lymphadenopathy reduced and he had gained 7 kg of weight. He displayed increased well-being and energy. The results are shown in Table 3.

The patient was given the weekly standard TFd treatment for a further six months. Three months after this treatment stopped, his condition remained stable. No relapses had occurred.

Example 5: Replication of HIV (HIV-1)-specific murine transfer factor using the LDV/7 cell line

HIV (HIV-1)-specific murine transfer factor prepared in accordance with Example 1 was filtered through a Milipore membrane (0.22 um). The filtrate was added to 40 ml of a culture of LDV/7 cells at a concentration of 5 x $10^5$ cells/ml and at a ratio of 1:1. The cells thus induced were maintained in culture for seven weeks at which time

- 14 -

the cell concentration had risen to $5 \times 10^9$ cells/ml, a sufficient number for extraction of further HIV (HIV-1)-specific transfer factor. The cells were then disrupted by repeated freeze-thawing and dialysed through a dialysis bag (Visking cellulose) against distilled water as described in Example 1.

Example 6: Treatment of patient (4)

Patient MB was a 26 year old female drug addict. She was suffering from ARC with polyadenopathy. When treatment started, her lymphocyte counts were:

| White blood cells (WBC) | 4800 |
| T4 lymphocytes | 499 (20%) |
| T8 lymphocytes | 899 |
| Total lymphocytes | 2496 |

Treatment consisted of a daily administration of $5 \times 10^7$ c.equ. of TFd for the first ten days. It was then followed by one dose of $5 \times 10^7$ c.equ./week. After six months of treatment, her lymphocyte counts were:

| $T_4$ lymphocytes | 1015 (40%) |
| T8 lymphocytes | 790 |
| Total lymphocytes | 2256 |
| WBC | 5200 |

The patient's condition had been stable for the next six months.

Example 7: Treatment of patient (5)

Patient XB was the twelve-month old child of patient MB of Example 6. The child was suffering from AIDS

- 15 -

contracted at birth. The child was treated with a daily administration of $5 \times 10^7$ c.equ. of TFd for the first ten days. It was then followed by one dose of $5 \times 10^7$ c.equ./week. After three months of treatment, the child's condition improved: increase of weight, decrease of the splenomegaly and of the adenopathies. The blood picture had stabilised within the normal range. Nine months further on, the child's condition is still stable with no signs of disease.

Example 8: Preparation and in vitro analysis of HIV (HIV-1) specific transfer factor derived from T4 and T8 lymphocytes from a human

Periphal blood lymphocytes were obtained from patient PAU of Example 3. These lymphocytes were extracted at a time when the patient was HIV-seropositive. The lymphocyte sub-populations T4 and T8 were separated out. Cell dialysates were obtained from each sub-population according to the procedure of Example 1. The HIV (HIV-1)-specific transfer factor thus obtained was replicated using the LDV/7 cell line as described in Example 5.

The respective HIV-specific transfer factors (cell dialysates) were assayed in vitro for their effect on HIV-1 replication by LDV/7 cells. It was found that the T4-derived dialysates (T4 TFd) increased the viral production of the LDV/7 cells three-fold, whereas the T8-derived dialysates (T8 TFd) increased viral production

0250234

- 16 -

only by 1.5 times, compared with the control. The control was a culture of HIV-1 infected LDV/7 cells incubated without dialysate.

**Example 9: Preparation of HIV (HIV-1) specific transfer factor derived from helper and suppressor lymphocyte sub-populations from mice**

Transfer factor was prepared by immunization of 10-14 week old Balb/c mice. The mice were given three bi-weekly subcutaneous injections of $10^7$ lymphocytes, from a pool obtained from four AIDS patients, mixed with 0.5 ml of complete Freund's Adjuvant. Six weeks after the first injection of lymphocytes, by which time the mice had achieved the necessary degree of immunisation, they were killed and their spleens removed. The helper and the suppressor lymphocyte sub-populations were separated out.

Transfer factor was derived from each lymphocyte sub-population as discussed in Example 1. In brief, the cells of each sub-population were disrupted by repeated freeze-thawing. The disrupted cells were dialysed through a dialysis bag (Visking cellulose) with a molecular weight cut-off of 12,000 against distilled water. The cell fractions thus-obtained constituted the helper-derived and suppressor-derived HIV-specific transfer factors. The transfer factors were replicated using the LDV/7 cell line in accordance with the procedure of Example 5.

**Example 10: Treatment of patient (6)**

Two AIDS patients suffering from Kaposi lesions were treated with T8 TFd. For the first ten days of treatment, each patient received daily $5 \times 10^7$ c.equ. of T8 TFd. Subsequently, each patient received $5 \times 10^7$ c.equ/week of T8 TFd. Under this treatment, the patients' lesions showed regression.

Two AIDS patients suffering from AIDS were treated with T4 TFd. Both received $5 \times 10^7$ c.equ. of T4 TFd per day. Each patient experienced a dramatic drop in their WBC count from 5000 to 600 within three days of beginning treatment. As a result, treatment with the T4 TFd was immediately stopped. One patient died within three days. The other patient recovered after daily administration of $5 \times 10^7$ c.equ. of T8 TFd. His WBC count increased within three days from 600 to 3000.

## Table 1  : PATIENT DUR

| Day | Treatment | Blood Tests | | | Original weight and change (kg) | Fever | Other symptoms and findings during treatment |
|-----|-----------|-------------|------|--------------------------|---------------------------------|-------|------------------------------------------------|
|     |           | T 4 | T4/T8 | Total lympho- cytes (µl) |  |  |  |
| 0 | (Admission) | 1029 | 1.56 | 2100 | | | Weight loss, fever, night sweats, dyspnea, pneumocystis carinii, fatigue, lymphadenopathy (5cm). |
| first month | Antibiotics and theophylline | | | | 56 (-6) | 39°5 | |
| third month | Sulphadiazine | | | | | 56-6 | Slight clinical improvement. |
| 124 | | 638 | 1.15 | 1890 | | 39°3 | |
| 140 | Standard TFd treatment | 726 | 1.06 | 2016 | | | |
| 147 | "      " | 632 | 0.87 | 1914 | (- 5) | 38.5 | Dyspnea ceases. Pneumocystis carinii not found. |
| 155 | "      " | 717 | 0.76 | 2240 | (- 5) | 38.5 | |
| 162 | "      " | 712 | 0.92 | 2035 | | | Night sweats. |
| 169 | "      " | 744 | 1.29 | 1860 | (- 5) | 37°8 | Decrease of asthenia; increased sense of well-being; decrease of lymphadenopathy (1 cm); skin-tests slightly positive for tetanus and steptococcus. |
| 176 | | 1401 | 1.73 | 2695 | | | Regained total muscle strength and went on vacation. |

Table 2 : PATIENT PAU

| Day | Treatment | Blood Tests | | | Original weight and change (kg) | Fever | Other symptoms and findings during treatment |
|---|---|---|---|---|---|---|---|
| | | T4 | T4/T8 | Total lympho-cytes (µl) | | | |
| 0 | (Admission) Sulphadiazine, sulphametho-xazole and trimethoprim. | | | | 66 (-5) | 38°9 | Fevers, pseudo-tumoral lymphadenopathy (lymph-nodes of 5 cm) and parotiditis, multiple facial "Kaposi-like" skin lesions; weight loss; fatigue; detection of HIV in the blood. |
| 35 | Patient discharged | | | | | 38° | |
| 102 | TFd $10^7$ c.equ./day x 13 days. | 1175 | 1.05 | 3264 | (- 7) | 38° | Fevers, weight loss, extreme fatigue, moderate lymphadenopathy. |
| 112 | | 1140 | 1.45 | 2375 | | | |
| 121 | | 562 | 1.14 | 1755 | | 37°7 | Facial lesions disappear; fevers cease; lymph-adenopathy decreases. HIV is not detected in the blood. |
| 133 | | 1252 | 0.95 | 3478 | | 37° | |
| 141 | | 1125 | 2.08 | 2250 | | 37° | |
| 147 | TFd $10^7$ c.equ. day x 15 days | | | | | | |
| 154 | | 1039 | 2.04 | 2210 | | | |
| 161 | | 1550 | 1.41 | 3444 | | | |
| 176 | Standard TFd treatment | 1061 | 1.53 | 2166 | | | |
| 198 | | 922 | 1.05 | 2144 | (- 1) | | Lymphadenopathy disappears. Patient's condition is excellent. He resumes normal activities. |
| 224 | | 1053 | 1.13 | 2394 | | | |

Table 3 : PATIENT KER

| Day | Treatment | Blood Tests | | | Original weight and change (kg) | Fever | Other symptoms and findings during t reatment |
|---|---|---|---|---|---|---|---|
| | | T 4 | T4/T8 | Total lympho-cytes (µl) | | | |
| -1½ Years | Amoxicillin | 81 | 0.06 | 2700 | | | ARC, fevers, lymphadenopathy, anti-HIV antibodies. |
| - 1 Year | " | | | | | | Polyadenopathy. Lymphocyte infiltration of the mucosa of the digestive tract and the peripheral nerves. |
| 0 | (Admission) Sulphadiazine, sulphametho-xazole and trimethoprim. | | | | 86 (-32) | 40° | Bronchitis, diarrhea, fevers, lymphadenopathy |
| 26 | | 515 | 0.50 | 2145 | (- 32) | 38° | |
| 37 | TFd $10^7$c.equ./day x 13 days | 390 | 0.18 | 3009 | (- 29) | 38° | |
| 51 | | 75 | 0.05 | 2494 | | 37° | Fevers subside; noticeable clinical improvement. |
| 57 | | 437 | 0.31 | 2150 | | | |
| 74 | | 158 | 0.06 | 3864 | | | |
| 85 | TFd $10^7$c.equ./day x 15 days | 302 | 0.12 | 3772 | | | |
| 92 | | 111 | 0.08 | 2765 | | | |
| 102 | | 154 | 0.07 | 3087 | | | |
| 113 | | 138 | 0.07 | 3450 | | | |
| 120 | Standard TFd treatment | 113 | 0.06 | 3763 | | | |
| 127 | " " | 121 | 0.07 | 3024 | | | Fatigue, but lymphadenopathy is reduced. |

Table 3 : PATIENT KER (Continued)

| Day | Treatment | Blood Tests | | | Original weight and change (kg) | Fever | Other symptoms and findings during treatment |
|-----|-----------|------|-------|-----------------------|----------------------------------|-------|-----------------------------------------------|
|     |           | T4   | T4/T8 | Total lympho-cytes (µl) |                                |       |                                               |
| 134 | Standard TFd treatment | 104 | 0.04 | 3456 |  | 37°8 |  |
| 141 | "      "   | 179 | 0.06 | 4484 |  |  | Fatigue ceases. Increased sense of well-being; weight gain 7 kg. |
| 148 | "      "   | 128 | 0.10 | 2560 | (- 24) | 37° |  |
| 155 | "      "   | 223 | 911  | 3186 |  |  | The spectacular improvement is confirmed. |

CLAIMS

1.    A process for the preparation of a product for use in combating an infection attributable to a retrovirus, which process comprises extracting cells of lymphoid origin and/or liver cells from an animal or human immunised with respect to the retrovirus, disrupting the cells and dialysing or filtering the contents of the cells thus-released thereby to obtain a retrovirus-specific transfer factor.

2.    A process according to claim 1, wherein the retrovirus is HTLV-I, HTLV-II or a Lentivirus.

3.    A process according to claim 2, wherein the retrovirus is a HIV, SIV or medi-visna virus.

4.    A process according to any one of the preceding claims, wherein the cells of lymphoid origin are, in the case of a human, peripheral blood lymphocytes or, in the case of an animal, spleen cells or cells from lymph nodes.

5.    A process according to any one of the preceding claims, wherein helper lymphocytes are disrupted and dialysed or filtered.

6.    A process according to any one of the preceding claims, further comprising replicating the retrovirus-specific transfer factor by means of a lymphoblastoid cell line.

7.    A process according to claim 6, wherein the cell line is the LDV/7 cell line.

8.    A process according to any one of the preceding claims, wherein the retrovirus-specific transfer factor is lyophilised.

9.    A product suitable for use in combating an infection attributable to a retrovirus, which product comprises a retrovirus-specific transfer factor derived from cells of lymphoid origin and/or liver cells of an animal or human immunised with respect to a retrovirus.

10.    A pharmaceutical composition comprising a pharmaceutically acceptable carrier or diluent and, as active ingredient, a retrovirus-specific transfer factor prepared from cells of lymphoid origin and/or liver cells of an animal or human immunised with respect to a retrovirus.

11.    A product containing

(a)    a retrovirus-specific transfer factor prepared from cells of lymphoid origin and or liver cells of an animal or human immunised with respect to a retrovirus and

(b)    one or more selected from thymic and/or spleen and/or liver extracts, anabolites and antiviral drugs

for simultaneous or sequential use in combating a viral infection attributable to a retrovirus in a human or animal.

European Patent Office

**EUROPEAN SEARCH REPORT**

Application number

EP 87305389.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP - A1 - 0 010 738 (A/S ALFRED BENZON) <br><br> * Abstract; claims 1-9; page 2, line 5 - page 3, line 20; page 8, lines 6-21 * | 1,2,8-10 | A 61 K 45/05 <br> A 61 K 35/14 <br> A 61 K 39/00 <br> A 61 K 35/26 <br> C 12 N 5/00 |
| X | DE - C2 - 2 064 296 (INSTITUT PASTEUR) <br><br> * Claims 1,4; page 2, lines 56-58; page 3, lines 1-4, 20-29; examples 1,4,5,7,8 * | 1,2,4, 6,8-10 | |
| D,X | GB - A - 1 592 954 (THE INT. INST. OFF DIFF. LTD) <br><br> * Claim 1; page 2, lines 85-127, especially lines 96-101 * | 1,4,6, 7,9,10 | |
| | | | **TECHNICAL FIELDS SEARCHED (Int. Cl.4)** |
| X | US - A - 3 991 182 (L.E. SPITLER et al.) <br><br> * Abstract; claim 1 * | 1,4,8, 9,10 | A 61 K 45/00 <br> A 61 K 35/00 <br> A 61 K 39/00 <br> C 12 N 5/00 <br> C 12 P 21/00 |
| X | US - A - 4 132 776 (W.S. JETER) <br><br> * Abstract; claims 1-7; column 3, line 60 - column 4, line 16; column 7, lines 4-10 * | 1,4,8-11 | |

The present search report has been drawn up for all claims

| Place of search | Date of completion of the search | Examiner |
|---|---|---|
| VIENNA | 27-08-1987 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

0250234

## DOCUMENTS CONSIDERED TO BE RELEVANT

EP 87305389.6

| Category | Citation of document with indication, where appropriate, of relevant passages | Relevant to claim | CLASSIFICATION OF THE APPLICATION (Int Cl 4) |
|---|---|---|---|
| D,X | GB - A - 1 443 948 (PIKTOR LTD) <br> * Claims 1-3; page 1, lines 12-88; page 2, lines 6-93; page 3, lines 85-93 * | 1,4,6, 8-10 | |
| A | EP - A1 - 0 165 120 (CENTRE NATIONAL DE LA RECH. SC.) <br> * Abstract; claims 1,6,7,13 * | 1,3,4, 6,9-11 | |
| A | EP - A1 - 0 051 216 (THE REGENTS OF THE UNIV. OF CALIFORNIA) <br> * Claims 1,5; example III * | 1,4-6, 9,10 | |
| A | US - A - 4 521 407 (R.W. PELHAM et al.) <br> * Abstract; claim 1 * | 1,3,9, 10 | |
| A | EP - A1 - 0 109 089 (A.A. GOTTLIEB) <br> * Claim 1; page 2, lines 12-18; page 3, line 9 - page 4, line 17 * | 1,4,9, 10 | |
| A | US - A - 4 180 627 (P.H. KLESIUS, H.H. FUDENBERG) <br> * Abstract; claim 1; column 1, line 64 - column 2, line 8; column 2, lines 39-47 * | 1,4,9, 10 | |

TECHNICAL FIELDS SEARCHED (Int Cl 4)

The present search report has been drawn up for all claims

| Place of search | Date of completion of the search | Examiner |
|---|---|---|
| VIENNA | 27-08-1987 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

0250234

| | DOCUMENTS CONSIDERED TO BE RELEVANT | | EP 87305389.6 |

| Category | Citation of document with indication, where appropriate, of relevant passages | Relevant to claim | CLASSIFICATION OF THE APPLICATION (Int. Cl.4) |
|---|---|---|---|
| A | <u>EP - A2 - 0 042 064</u> (A.A. GOTTLIEB) <br> * Abstract; claims 1,10 * <br> ---- | 1,4,9, 10 | |

TECHNICAL FIELDS
SEARCHED (Int. Cl.4)

The present search report has been drawn up for all claims

| Place of search <br> VIENNA | Date of completion of the search <br> 27-08-1987 | Examiner <br> MAZZUCCO |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on. or after the filing date
D : document cited in the application
L : document cited for other reasons

& : member of the same patent family, corresponding document

EPO Form 1503 03 82